# EUROPÄISCHE PATENTSCHRIFT

(11) **EP 2 054 048 B1**
(45) Veröffentlichungstag und Bekanntmachung des Hinweises auf die Patenterteilung: **28.07.2010**
(21) Anmeldenummer: 07786700.0
(22) Anmeldetag: 17.08.2007
(51) Int. Cl.: A61K 31/135, C07C 211/42

(54) **NEUE SALZE DES WIRKSTOFFS RASAGILIN**
NOVEL SALTS OF THE ACTIVE SUBSTANCE RASAGILINE
NOUVEAUX SELS DE L'AGENT ACTIF RASAGILINE

(30) Priorität: 18.08.2006 EP 06017286
(43) Veröffentlichungstag der Anmeldung: 06.05.2009
(73) Patentinhaber: Ratiopharm GmbH, 89079 Ulm (DE)
(72) Erfinder: STAHL, P., Heinrich, 79104 Freiburg (DE)
(74) Vertreter: Best, Michael
(86) Internationale Anmeldenummer: PCT/EP2007/007296
(87) Internationale Veröffentlichungsnummer: WO 2008/019871

(56) Entgegenhaltungen:
- WO-A-95/11016
- GOULD P L: "SALT SELECTION FOR BASIC DRUGS" INTERNATIONAL JOURNAL OF PHARMACEUTICS, AMSTERDAM, NL, Bd. 33, Nr. 1/3, 1986, Seiten 201-217, XP002074725 ISSN: 0378-5173

## Beschreibung

Die Erfindung betrifft neue Salze des Wirkstoffs Rasagilin. Die Salze zeigen auch nach längerer Lagerzeit eine ausgezeichnete Verarbeitbarkeit und nach der Verarbeitung in Tabletten eine ausgezeichnete, den bekannten Salzen in der Regel überlegene Lagerstabilität.

Rasagilin, das R(+)-Enantiomer von n-Propargyl-1-aminoindan, ist ein seit langem bekannter Wirkstoff, der insbesondere zur Behandlung von Parkinson, Demenz und Alzheimer eingesetzt wird. Weitere Indikationen sind z.B. in der EP-A 436 492 und der WO 95/11016 beschrieben, auf die diesbezüglich ausdrücklich Bezug genommen wird. Die Verbindung ist bereits von der Offenbarung der DE 1 443 559 und der DE 1 443 403 umfasst. Als Einzelverbindung ist Rasagilin in der EP-A 436 492 beschrieben, die Rasagilin und allgemein pharmazeutisch verträgliche Säureadditionssalze davon offenbart, spezifisch das Hydrochlorid und das Tartrat des Rasagilins. Weitere Salze des Rasagilins werden in der WO 95/11016 beschrieben, nämlich das Sulfat, Phosphat, Mesylat (Methansulfonat), Maleat, Esylat (Ethansulfonat), Acetat, Fumarat, Hydrobromid, Tosylat (Toluolsulfonat) und Benzoat.

Die bekannten Salze fallen als weißes Kristallpulver an, zeigen aber insbesondere nach längerer Lagerung Nachteile. So weist beispielsweise das Tartrat des Rasagilins bereits unmittelbar nach der Herstellung unerwünschte Agglomerate auf, die die Verarbeitung erschweren. Das Mesylat, Hydrochlorid und Phosphat des Rasagilins zeigen derartige Agglomerate nach Lagerung selbst in einer geschlossenen Glasflasche und können auch eine leichte Verfärbung zeigen. Bei Lagerung unter feuchten Bedingungen (40°C, 75% relative Feuchte in offener Glasflasche) bildet sich aus dem Mesylat, dem Hydrochlorid und dem Phosphatsalz des Rasagilins ein klebriges Pulver, das nur schwer zu verarbeiten ist. Agglomerate sind bei der Verarbeitung des Wirkstoffs störend, insbesondere bei der Verarbeitung zu festen Arzneimitteln, da sie zu Inhomogenitäten in dem Arzneimittel führen können. Vor allem auch bei der Herstellung von Tabletten durch Direktverpressung treten Schwierigkeiten auf, wenn der Wirkstoff teilweise agglomeriert ist.

Die bekannten Rasagilinsalze sind zwar in festen Arzneimitteln weitgehend lagerstabil, eine gewisse Zersetzung des Wirkstoffs tritt aber dennoch auf. Außerdem zeigen viele Salze eine unerwünschte Hygroskopizität.

In der Technik besteht ein Bedarf nach Salzen des Rasagilins, die eine verbesserte Lagerstabilität aufweisen und die daher auch nach längerer Lagerung noch gut verarbeitet werden können. In festen Arzneimittelformulierungen sollen die Salze chemisch mindestens so stabil sein, wie die bekannten Salze, bevorzugt eine höhere Stabilität aufweisen und eine möglichst geringe Hygroskopizität zeigen.

Entsprechend werden erfindungsgemäß Salze des Rasagilins mit einer Säure der allgemeinen Formel I 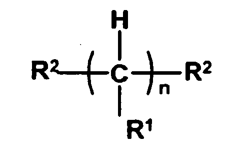 zur Verfügung gestellt, in der die Reste R¹ unabhängig voneinander ein Wasserstoffatom oder einen C₁-C₄-Alkylrest darstellen, R² eine COOH-Gruppe oder eine S(O)ₘH-Gruppe ist, n eine ganze Zahl im Bereich von 0 bis 4 ist und m den Wert 2, 3 oder 4 hat.

Erfindungsgemäß bevorzugt handelt es sich bei den Resten R¹ unabhängig voneinander um Wasserstoffatome oder Methylgruppen, am stärksten bevorzugt handelt es sich bei allen Resten R¹ um Wasserstoffatome. Der Index m ist erfindungsgemäß bevorzugt 3, so dass es sich erfindungsgemäß bevorzugt bei dem Rasagilinsalz um ein Disulfonatsalz handelt. Der Index n ist bevorzugt 0 oder 2. Wenn R² eine S(O)ₘH-Gruppe ist, ist n bevorzugt nicht 0. Ganz besonders bevorzugt sind erfindungsgemäß das Rasagilin-Oxalat und das Rasagilin-Edisilat (= Rasagilin-Ethandisulfonat, Salz der Ethandisulfonsäure), und am stärksten bevorzugt ist erfindungsgemäß das Rasagilin-Edisilat

Erfindungsgemäß kann es sich bei den Salzen des Rasagilins um Monosalze oder um Disalze oder um Gemische aus Mono- und Disalzen handeln, das heißt eine oder beide Säuregruppen der erfindungsgemäßen Disäuren bilden ein Salz mit einem Rasagilin-Molekül. Falls freie Säuregruppen vorhanden sind, können diese gegebenenfalls Salze mit weiteren üblichen pharmazeutisch verträglichen Gegenionen wie Natrium, Kalium, etc. bilden oder als freie Säuregruppen vorliegen. Bevorzugt sind erfindungsgemäß aber die Dicarbonsäuresalze bzw. die Disulfonsäuresalze des Rasagilins.

Überraschend wurde gefunden, dass die Rasagilinsalze, insbesondere das Rasagilin-Oxalat und das Rasagilin-Edisilat, als weißes Kristallpulver vorliegen, das frei von Agglomeraten ist und auch nach langer Lagerzeit keine Agglomerate bildet. Dies gilt vor allem bei trockener Lagerung aber insbesondere bei dem Rasagilin-Edisilat auch bei Lagerung unter feuchten Bedingungen. Dies ermöglicht eine leichtere Verarbeitung der erfindungsgemäßen Rasagilinsalze, vor allem auch bei der Herstellung von festen Arzneimitteln wie Tabletten und vor allem bei der Herstellung von Tabletten durch Direkttablettierung.

Die Erfindung betrifft damit auch Tabletten, die durch Direkttablettierung hergestellt werden, aber auch Granulate und Tabletten, die durch eine übliche Granulationsmethode oder durch Kompaktierung hergestellt werden.

Ein weiterer Vorteil der erfindungsgemäßen Rasagilinsalze ist ihre deutlich unterschiedliche Löslichkeit. So ist das Rasagilin-Edisilat sehr schnell und gut löslich, was es besonders vorteilhaft zur Formulierung von schnell-freisetzenden, festen Arzneiformen, insbesondere von schnell-freisetzenden Tabletten, macht. Demgegenüber ist beispielsweise das Rasagilin-Oxalat erheblich schwerer löslich, so dass das Rasagilin-Oxalat bevorzugt für langsam-freisetzende oder verzögert-freisetzende Arzneimittel verwendet werden kann. Erfindungsgemäß können die Arzneimittel mit den neuen Rasagilinsalzen sowohl schnell-freisetzende als auch verzögernd- oder hinhaltend-freisetzende Arzneimittel sein. Bevorzugt sind schnell-freisetzende Arzneimittel.

Die erfindungsgemäßen Rasagilinsalze können z.B. hergestellt werden, indem man Rasagilin-Base in einem geeigneten Lösemittel löst oder suspendiert und mit einer Lösung oder Suspension der entsprechenden Disäure versetzt. Durch Stehenlassen und gegebenenfalls Kühlen fällt das Rasagilinsalz aus, das dann auf übliche Art und Weise weiterverarbeitet werden kann. Geeignete Lösemittel sind z.B. organische Lösemittel oder Lösemittelgemische, in denen die Säure löslich ist und das Rasagilin löslich ist und das Rasagilinsalz unlöslich ist, oder Lösemittel, in denen entweder die Säure oder das Rasagilin unlöslich ist und das Rasagilinsalz bei höherer Temperatur löslich und bei niedriger Temperatur unlöslich ist. Geeignete Lösemittel sind z.B. polarprotische Lösemittel wie Alkohole, insbesondere C₁-C₈-Alkanole wie C₁-C₄-Alkanole, wobei 2-Propanol besonders bevorzugt ist. Durch diese Verfahren fallen die Salze kristallin an.

Werden Rasagilinsalze auf die vorstehende Weise hergestellt, fallen sie sehr feinteilig und mit enger Größenverteilung an. Insbesondere das erfindungsgemäß bevorzugte Rasagilin-Edisilat fällt sehr feinteilig an. Bei den so hergestellten Salzen, insbesondere dem erfindungsgemäßen Rasagilin-Edisilat und dem erfindungsgemäßen Rasagilin-Oxalat, aber auch den nicht erfindungsgemäßen bekannten Rasagilinsalzen wie dem Rasagilin-Tartrat und dem Rasagilin-Mesylat, ist der Anteil an Teilchen mit einer Größe von 250 µm oder darunter in der Regel bei über 90%, bevorzugt bei über 95%. Bevorzugte Teilchengrößenverteilungen für die erfindungsgemäßen Salze, aber auch für die nicht erfindungsgemäßen Tartrate und Mesylate sind wie folgt:

| **d(0.1) [µm]** | **d(0.5) [µm]** | **d(0.9) [µm]** | |
|---|---|---|---|
| 1,0 - 40 | 5,0 - 70 | 10 - 200 | üblich |
| 1,5 - 30 | 7,5 - 60 | 20 - 180 | bevorzugt |
| 2 - 25 | 10 - 55 | 30 - 170 | stärker bevorzugt |

wobei die Teilchengrößen für das Edisilat und das Tartrat eher an den unteren Grenzwerten liegen und die Teilchengrößen für das Oxalat und das Mesylat eher an den oberen Grenzwerten liegen. Die Bestimmung der Teilchengrößen erfolgt wie in den Beispielen beschrieben.

Die erfindungsgemäßen Rasagilinsalze können ebenfalls amorph hergestellt werden, z.B. durch Sprühtrocknung. Auch die amorphen Salze sind Gegenstand der Erfindung. Bevorzugt sind kristalline Salze.

Die Rasagilin-Base kann auf bekannte Art und Weise hergestellt werden, beispielsweise wie es in der EP-A 436 492 beschrieben ist.

Die erfindungsgemäßen Rasagilinsalze werden auf an sich bekannte Art und Weise zu Arzneimitteln, insbesondere festen Arzneimitteln, verarbeitet. In den Arzneimitteln ist das erfindungsgemäße Rasagilinsalz in einer therapeutisch wirksamen Menge vorhanden, bei einer festen Arzneiform wie einer Tablette, insbesondere in einer Menge von etwa 0,1 mg bis etwa 1000 mg pro Dosiseinheit (d.h. z.B. pro Tablette), bevorzugt in einer Menge von etwa 0,5 mg bis etwa 10 mg pro Dosiseinheit. Im Fall einer flüssigen Dosierungsform enthält ein erfindungsgemäßes Arzneimittel ebenfalls eine therapeutisch wirksame Menge des erfindungsgemäßen Rasagilinsalzes, insbesondere eine Menge von etwa 0,1 mg/ml bis etwa 100 mg/ml, bevorzugt etwa 0,5 mg/ml bis etwa 10 mg/ml des Arzneimittels. Eine bevorzugte verabreichte Menge des Arzneimittels liegt erfindungsgemäß im Bereich von 0,1 ml bis 1,0 ml des Arzneimittels pro Tag.

Erfindungsgemäß bevorzugt sind Festdosierungsformen wie Pellets, Granulate, Satchets, Hartgelantinekapseln, Weichgelantinekapseln, Dragees, Tabletten, etc. Besonders bevorzugt sind erfindungsgemäß Tabletten, die beschichtet oder unbeschichtet sein können. Die Tabletten können erfindungsgemäß durch übliche Granulationsverfahren oder bevorzugt durch Direktverpressen hergestellt werden. Die Formulierung der erfindungsgemäßen Rasagilinsalze erfolgt mit üblichen pharmazeutischen Hilfs- und Zusatzstoffen. Geeignete Zusatzstoffe sind in der Regel Füllstoffe, Bindemittel, Sprengmittel, Schmiermittel, Stabilisatoren und Fließreguliermittel, sowie gegebenenfalls weitere Zusatzstoffe.

Die erfindungsgemäß bevorzugten festen Dosierungsformen, insbesondere die erfindungsgemäßen Tabletten enthalten in der Regel mehr als 50 Gew.-% Füllstoffe, stärker bevorzugt mehr als 65 Gew.-% Füllstoffe, besonders bevorzugt 70 bis 95 Gew.-% Füllstoffe.

Der Gehalt an Sprengmittel ist in der Regel 1 bis 25 Gew.-%, vorzugsweise 1 bis 20 Gew.-%, insbesondere 2 bis 15 Gew.-%. Geeignete Bereiche für den Gehalt an Sprengmittel sind auch z.B. 2 bis 5 Gew.-% oder 15 bis 20 Gew.-%, in Abhängigkeit der verwendeten Sprengmittel, Füllstoffe und übrigen Zusatzstoffe.

Der Gehalt an Schmiermittel ist in der Regel 0,1 bis 4 Gew.-%, vorzugsweise 0,2 bis 4 Gew.-%.

Falls die Zusammensetzung ein Fließregulierungsmittel umfasst, ist dieses in der Regel in einer Menge von 0,5 bis 5 Gew.-%, vorzugsweise 1 bis 4 Gew.-%, insbesondere 2 bis 3 Gew.-% vorhanden.

Der Gehalt an Stabilisator (falls vorhanden) ist in der Regel im Bereich von 0,5 bis 4 Gew.-%, bevorzugt 1 bis 3 Gew.-%.

Als Füllstoffe können eine oder mehrere Verbindungen verwendet werden, die einen Teil des Materials zum Erreichen der erforderlichen und gewünschten Tablettengesamtmasse liefern. Einsetzbar sind unter anderem mikrokristalline Cellulose in verschiedenen Partikelgrößen, insbesondere mit einer mittleren Partikelgröße im Bereich von 20 µm bis 200 µm, insbesondere im Bereich von 50 µm bis 150 µm, wie z.B. etwa 100 µm, wie die bekannten Avicel-Produkte wie Avicel PH-101 und PH-102. Weitere geeignete Füllstoffe sind z.B. Lactose, Cellactose (eine Mischung aus Cellulose und Lactose), Calciumphosphat, Dextrose, Mannit, Maltodextrin, Isomalt, gegebenenfalls auch Sorbit und Saccharose. Falls eine Direktverpressung vorgesehen ist, sollte bei der Auswahl der Füllstoffe darauf geachtet werden, dass Qualitäten verwendet werden, die für die Direktverpressung von Tabletten geeignet sind. Dies wird bei den kommerziellen Produkten jeweils vom Hersteller angegeben bzw. kann durch einfache Versuche überprüft werden. Das am stärksten bevorzugte Füllmittel ist mikrokristalline Cellulose (Handelsprodukte sind beispielsweise Avicel, Vivapur und Emcocel).

Neben den vorstehenden Füllmitteln sind insbesondere auch Mannit, Sorbit und Isomalt als Füllmittel bevorzugt, ganz besonders bevorzugt ist erfindungsgemäß das Füllmittel Mannit. Hier kann beispielsweise auf die Produkte Pearlitol verwiesen werden.

Erfindungsgemäß bevorzugt ist als Füllmittel auch ein Gemisch aus mikrokristalliner Cellulose und Mannit. Das Verhältnis von mikrokristalliner Cellulose und Mannit ist bei dieser Ausführungsform nicht besonders eingeschränkt, bevorzugt wird aber mehr Mannit eingesetzt als mikrokristalline Cellulose, und das Verhältnis ist bevorzugt im Bereich von 1:1,1 bis 1:5, stärker bevorzugt im Bereich von 1:2 bis 1:4.

Geeignete Sprengmittel sind im Stand der Technik bekannt. Sprengmittel werden häufig auch mit dem englischen Begriff "Disintegrants" bezeichnet. Erfindungsgemäß bevorzugte Sprengmittel sind z.B. Crospovidone (Kollidon CL) und Stärke bzw. vorverkleisterte Stärke, insbesondere das Handelsprodukt "Starch 1500". Weitere geeignete Stärken sind z.B. unter den Bezeichnungen Lycatab PGS, Prejel und Sepistab ST 200 kommerziell erhältlich. Weiterhin können auch die bekannten sogenannten "Super Disintegrants" verwendet werden, wie Croscarmellose Natrium (z.B. Ac-Di-Sol, u.a.) und Carboxymethylstärke Natrium (z.B. Explotab, Primojel, u.a.). Besonders bevorzugt sind Stärken wie Starch 1500.

Erfindungsgemäß kann die Zusammensetzung als Schmiermittel eine oder mehrere Verbindungen enthalten, die die Herstellung und die Verarbeitung der Tablette unterstützen. Verwendbare Schmiermittel sind unter anderem Stearinsäure und deren Derivate, wie Calciumstearat, und insbesondere Natriumstearylfumarat (das z.B. unter der Bezeichnung Pruv kommerziell erhältlich ist) und Magnesiumstearat, Glycerolmono-, -di- und insbesondere -tristearat, hydriertes Pflanzenöl (z.B. Lubritab, Dynasan, Sterotext) oder ein Polyethylenglykol (z.B. Lutrol, Carbowax).

Optional kann die erfindungsgemäße pharmazeutische Zusammensetzung ein oder mehrere Fließregulierungsmittel umfassen. Geeignete Fließregulierungsmittel sind Magnesiumtrisilikat, Talk und insbesondere Siliciumdioxid (z.B. Aerosil).

Die erfindungsgemäßen pharmazeutischen Zusammensetzungen können ebenfalls noch Stabilisatoren für den Wirkstoff enthalten, wie Zitronensäure, Weinsäure, Milchsäure, etc., bevorzugt Zitronensäure.

Die erfindungsgemäßen pharmazeutischen Zusammensetzungen können weitere übliche pharmazeutisch verträgliche Zusatz- und Hilfsstoffe enthalten, bevorzugt enthalten sie aber außer den vorstehend angegebenen (Füllstoff, Sprengmittel, Schmiermittel und gegebenenfalls Fließregulierungsmittel und Stabilisator) keine weiteren Hilfsstoffe.

Einige Füllstoffe wie mikrokristalline Cellulose können auch als Bindemittel dienen. Auch Füllstoffe mit Bindemittelfunktion zählen daher im Rahmen dieser Anmeldung zu den Füllstoffen.

Liegt die erfindungsgemäße pharmazeutische Zusammensetzung als Tablette vor, kann sie mit einem oder mehreren Beschichtungsmitteln filmbeschichtet sein. Verwendbare Beschichtungsmittel sind Hypromellose (Hydroxypropylmethylcellulose), Polyvinylalkohol, Natriumcarboxymethylcellulose und verschiedene Methacrylsäurepolymere (Eudragite), wobei Hypromellose und insbesondere Eudragite bevorzugt sind. Das Beschichten der Tabletten erfolgt auf übliche Art und Weise. In der Beschichtung können außer dem Beschichtungsmittel weitere übliche Bestandteile von Tablettenbeschichtungen wie Weichmacher, Pigmente, Porenbildner oder Suspensionsstabilisatoren vorhanden sein wie z.B. Polyethylenglykol (PEG), Talk oder Titandioxid und gegebenenfalls auch Lactose.

Das Tablettengewicht ist nicht besonders eingeschränkt, üblich sind Tabletten mit 100 bis 600 mg, z.B. 100 bis 300 mg, insbesondere etwa 200 mg.

Die folgenden Beispiele erläutern die Erfindung. Die eingesetzte Rasagilin-Base wurde nach der Vorschrift der EP-A 436 492 erhalten.

### Beispiel 1

### Rasagilin-Edisilat

47 g Rasagilin-Base wurden in 668 ml 2-Propanol suspendiert. Anschließend wurden 25,9 g Ethandisulfonsäure gelöst in 668 ml 2-Propanol zugegeben. Aus der zwischenzeitlich klaren Lösung fiel ein weißer Feststoff aus. Die Suspension wurde 2 Tage im Kühlschrank stehengelassen, danach filtriert und mit 100 ml gekühltem 2-Propanol nachgewaschen. Der Rückstand wurde im Trockenschrank bei 45°C getrocknet. Ausbeute: 54,9 g (56,3% der Theorie) weiße Kristalle; Wassergehalt (Karl Fischer): ca. 0,1%; Schmelzpunkt 201°C (klare, durchsichtige Schmelze); DSC: Peak bei 208,83°C; [α]_{D}²⁰: +40,3° (2% in H₂O); Elementaranalyse: C = 58,76% (Theorie: 58,63%), H = 6,16% (Theorie: 6,05%), N = 5,13% (Theorie: 5,26%), S = 12,22% (Theorie: 12,04%).

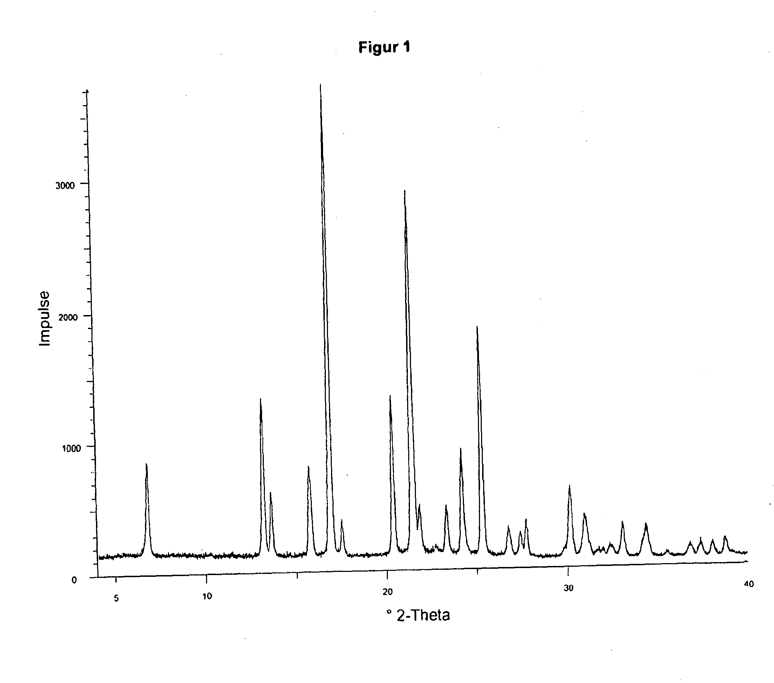

Von dem hergestellten Salz wurde ein Pulverröntgendiagramm erstellt, das in Figur 1 gezeigt ist. Eine Liste der 2-Thetawerte ist wie folgt:

| 2-Theta | d-Werte | I (rel) |
|---|---|---|
| 6,79° | 13,015 | 22,9 |
| 13,15° | 6,726 | 35,7 |
| 13,60° | 6,503 | 16,5 |
| 15,75° | 5,621 | 21,5 |
| 16,90° | 5,241 | 100,0 |
| 17,54° | 5,053 | 10,4 |
| 20,38° | 4,355 | 35,7 |
| 21,45° | 4,140 | 77,6 |
| 21,83° | 4,067 | 13,0 |
| 22,70° | 3,915 | 5,0 |
| 23,32° | 3,811 | 13,0 |
| 24,19° | 3,676 | 24,5 |
| 25,31° | 3,516 | 49,7 |
| 26,76° | 3,328 | 8,6 |
| 27,41° | 3,252 | 7,2 |
| 27,74° | 3,214 | 9,7 |
| 29,79° | 2,997 | 3,4 |
| 30,17° | 2,960 | 16,5 |
| 30,98° | 2,884 | 10,5 |
| 31,64° | 2,825 | 3,6 |
| 31,93° | 2,801 | 3,3 |
| 32,36° | 2,764 | 4,6 |
| 33,07° | 2,706 | 8,8 |
| 34,13° | 2,625 | 5,1 |
| 34,37° | 2,607 | 8,4 |
| 35,50° | 2,527 | 2,8 |
| 36,80° | 2,440 | 4,4 |
| 37,39° | 2,403 | 5,0 |
| 38,04° | 2,363 | 4,2 |
| 38,74° | 2,322 | 5,3 |
| 40,04° | 2,250 | 2,0 |
| 40,72° | 2,214 | 2,3 |
| 41,26° | 2,186 | 3,2 |
| 41,48° | 2,175 | 2,5 |
| 42,07° | 2,146 | 2,5 |
| 42,91° | 2,106 | 4,0 |
| 43,67° | 2,071 | 2,6 |
| 44,02° | 2,055 | 2,6 |
| 44,94° | 2,015 | 5,4 |
| 46,42° | 1,955 | 2,1 |
| 46,90° | 1,936 | 2,1 |
| 48,43° | 1,878 | 3,0 |
| 48,86° | 1,863 | 3,2 |
| 49,19° | 1,851 | 2,3 |
| 50,08° | 1,820 | 2,4 |
| 50,94° | 1,791 | 2,5 |
| 51,56° | 1,771 | 2,7 |
| 51,98° | 1,758 | 2,7 |
| 53,34° | 1,716 | 2,1 |
| 53,98° | 1,697 | 1,9 |
| 58,66° | 1,573 | 1,6 |
| 59,37° | 1,555 | 1,8 |
| 59,73° | 1,547 | 1,8 |
| 61,10° | 1,516 | 1,7 |
| 63,36° | 1,467 | 1,8 |
| 66,19° | 1,411 | 1,8 |
| 66,83° | 1,399 | 1,5 |
| 69,32° | 1,354 | 1,5 |
| 71,58° | 1,317 | 1,5 |
| 72,10° | 1,309 | 1,6 |
| 74,48° | 1,273 | 1,4 |
| 77,89° | 1,225 | 1,5 |
| 79,53° | 1,204 | 1,4 |

Die 2-Thetawerte können eine gewisse Abweichung von ± 0,2° aufweisen, und es wird angenommen, dass die erhaltene polymorphe Form des Rasagilin-Edisilats durch die fünf intensitätsstärksten Peaks bevorzugt durch die sieben intensitätsstärksten Peaks, stärker bevorzugt durch die zehn intensitätsstärksten Peaks eindeutig charakterisiert wird.

### Beispiel 2

### Rasagilin-Oxalat

60 g Rasagilin-Base wurden in 885 ml 2-Propanol suspendiert. Anschließend wurden 32,7 g Oxalsäure gelöst in 885 ml 2-Propanol zugegeben. Das Gemisch wurde über Nacht im Kühlschrank stehengelassen, danach filtriert und mit 100 ml gekühltem 2-Propanol nachgewaschen. Der Rückstand wurde im Trockenschrank bei 45°C getrocknet. Ausbeute: 87,6 g (93,1% der Theorie) weiße Kristalle; Wassergehalt (Karl Fischer): ca. 0,1%; Schmelzpunkt 204°C (klare, braune Schmelze); DSC: Peak bei 209,52°C; [α]_{D}²⁰: +28,9° (2% in Ethanol); Elementaranalyse: C = 64,39% (Theorie: 64,36%), H = 5,91% (Theorie: 5.79%), N = 5,43% (Theorie: 5,36%).

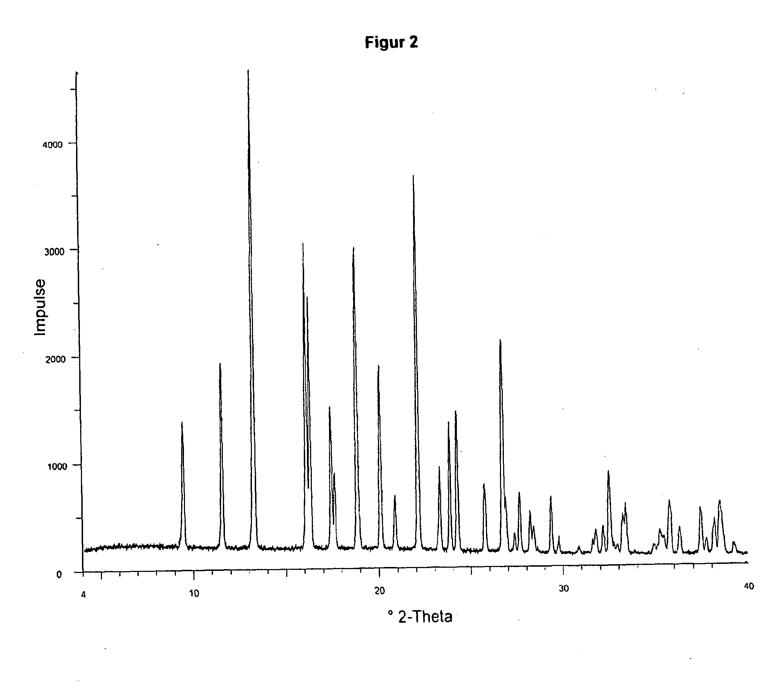

Von dem erhaltenen Produkt wurde ein Pulverröntgendiagramm aufgenommen, das in Figur 2 gezeigt ist. Die Peakliste (bis zu einem 2-Thetawert von 40,09) ist wie folgt:

| 2-Theta | d-Werte | I (rel) |
|---|---|---|
| 9,43° | 9,373 | 25,0 |
| 11,55° | 7,657 | 38,8 |
| 13,27° | 6,667 | 100,0 |
| 16,31° | 5,430 | 52,5 |
| 17,44° | 5,081 | 29,3 |
| 17,63° | 5,027 | 15,7 |
| 18,85° | 4,704 | 63,0 |
| 20,10° | 4,414 | 38,3 |
| 20,84° | 4,259 | 11,6 |
| 22,11° | 4,017 | 77,8 |
| 23,32° | 3,811 | 17,6 |
| 23,86° | 3,727 | 26,4 |
| 24,27° | 3,664 | 28,9 |
| 25,75° | 3,457 | 14,1 |
| 26,75° | 3,330 | 43,7 |
| 26,96° | 3,309 | 11,3 |
| 27,37° | 3,256 | 3,9 |
| 27,64° | 3,225 | 12,6 |
| 28,21° | 3,161 | 8,5 |
| 28,38° | 3,142 | 5,4 |
| 29,39° | 3,036 | 11,6 |
| 29,78° | 2,998 | 3,3 |
| 30,86° | 2,896 | 1,7 |
| 31,60° | 2,829 | 3,0 |
| 31,78° | 2,813 | 5,0 |
| 32,19° | 2,779 | 5,5 |
| 32,53° | 2,750 | 17,2 |
| 32,78° | 2,730 | 2,3 |
| 32,96° | 2,716 | 2,1 |
| 33,26° | 2,692 | 8,1 |
| 33,43° | 2,679 | 10,0 |
| 33,91° | 2,642 | 0,7 |
| 34,95° | 2,565 | 1,9 |
| 35,23° | 2,545 | 5,2 |
| 35,46° | 2,529 | 3,7 |
| 35,79° | 2,507 | 11,0 |
| 36,32° | 2,471 | 5,5 |
| 37,42° | 2,402 | 9,7 |
| 37,76° | 2,380 | 3,3 |
| 38,18° | 2,355 | 7,1 |
| 38,49° | 2,337 | 10,9 |
| 39,21° | 2,296 | 2,3 |
| 40,09° | 2,247 | 0,8 |

Die 2-Thetawerte können eine Abweichung von ± 0,2° aufweisen, und es wird angenommen, dass die polymorphen Form des Rasagilin-Oxalats durch die fünf intensitätsstärksten Peaks, bevorzugt durch die sieben intensitätsstärksten Peaks, stärker bevorzugt durch die zehn intensitätsstärksten Peaks und insbesondere durch die 15 intensitätsstärksten Peaks der vorstehenden Liste eindeutig charakterisiert wird.

Die Röntgenpulverdiagramme wurden wie folgt aufgenommen:
Die Messungen erfolgten in Transmissionsgeometrie.

### Probenpräparation:

Die Probe wird an Luft im Achatmörser gemörsert. Die gemörserte Substanz wird zwischen zwei Folien in die Probenhalterung eingespannt.

### Messparameter:

Trägerfolie: reflexfreie Polyacetat-Folie
Messbereich 2θ = 4° - 90°
PSD step 0,5°
Messzeit 60 s/step entsprechend 840 s pro Messpunkt
Probenrotation

### Geräteparameter:

Transmissionsdiffraktometer Stoe Stadi P, Baujahr 2004
Strahlung: Cu Kα₁; λ = 1,5406 Å
Monochromator: vorgestellter gebogener Ge(111)-Monochromator
Divergenzblende: 6 mm (vertikal)
Kurzkollimator mit horizontaler Divergenzblende 1 mm
ortsempfindlicher Detektor (linearer PSD; Winkelauflösung besser als 0,06° 2θ FWHM)

### Generatoreinstellung:

40 kV, 30 mA.

### Beispiel 3

### Bestimmung der Löslichkeit

0,5 g Rasagilin-Oxalat bzw. 3 g Rasagilin-Edisilat wurden mit jeweils 5 ml des jeweiligen Lösungsmittels in ein 25 ml Becherglas gegeben und ultrabeschallt. Nach einstündiger Lagerung bei 37°C wurden die Proben filtriert, die Filtrate verdünnt und mittels HPLC analysiert. Außerdem wurde der pH-Wert der Lösungen bestimmt.

| **Salz** | **Lösungsmittel** | **pH-Wert der gesättigten Lösung** | **Löslichkeit** **[mg/ml]** |
|---|---|---|---|
| Rasagilin-Edisilat | 0,1 N HCl | 1,0 | 359,8 |
| | USP Acetatpuffer, pH 4,5 | 4,4 | 475,3 |
| | USP Phosphatpuffer, pH 6,8 | 6,2 | 497,7 |
| | Demin. Wasser | 4,4 | 342,5 |
| Rasagilin-Oxalat | 0,1 N HCl | 1,2 | 29,9 |
| | USP Acetatpuffer, pH 4,5 | 3,4 | 24,3 |
| | USP Phosphatpuffer, pH 6,8 | 3,4 | 24,2 |
| | Demin. Wasser | 2,7 | 19,7 |

### Beispiel 4

### Bestimmung der Hydroskopizität

Rasagilin-Edisilat gemäß Beispiel 1, Rasagilin-Oxalat gemäß Beispiel 2 und Rasagilin-Mesylat, das in dem derzeit kommerziell erhältlichen Rasagilin-Arzneimittel vorhanden ist, wurden in Feuchtekammern bei definierter Luftfeuchtigkeit und Raumtemperatur gelagert. Bei Lagerung unter 93% Luftfeuchtigkeit nahm das Rasagilin-Mesylat große Mengen Wasser auf und verflüssigte sich. Die erfindungsgemäßen Rasagilinsalze zeigten keinerlei Wasseraufnahme.

### Beispiel 5

### Stabititätsuntersuchungen

Rasagilin-Edisilat und -Oxalat, sowie -Mesylat, -Hydrochlorid, -Phosphat und -Tartrat wurden mehrere Wochen unter Stressbedingungen eingelagert. Nach festgelegten Zeitintervallen wurden Aussehen, chemische Reinheit und Wassergehalt untersucht.

Lagerung bei 60°C in geschlossener Glasflasche:

| **Zeitpunkt** | **Edisilat** | **Oxalat** | **Mesylat** | **Hydrochlorid** | **Phosphat** | **Tartrat** |
|---|---|---|---|---|---|---|
| **0** | weißes, kristallines Pulver | weißes, kristallines Pulver | weißes, kristallines Pulver | weißes, kristallines Pulver | weißes, kristallines Pulver | weißes, kristallines Pulver mit Agglomeraten |
| **4 Wochen** | unverändert | unverändert | weißes Pulver, Agglomerate | weißes Pulver, Agglomerate | gelbes Pulver, Agglomerate | unverändert |
| **8 Wochen** | unverändert | unverändert | weißes Pulver, Agglomerate | weißes Pulver, Agglomerate | gelbes Pulver, Agglomerate | unverändert |
| **12 Wochen** | unverändert | unverändert | weißes Pulver, Agglomerate | weißes Pulver, Agglomerate | gelbes Pulver, Agglomerate | unverändert |

Chemische Reinheit und Wassergehalt blieben bei allen Proben unverändert.

Lagerung bei 40°C/75% relative Feuchte in offener Glasflasche:

| **Zeitpunkt** | **Edisilat** | **Oxalat** | **Mesylat** | **Hydrochlorid** | **Phosphat** | **Tartrat** |
|---|---|---|---|---|---|---|
| **0** | weißes, kristallines Pulver | weißes, kristallines Pulver | weißes, kristallines Pulver | weißes, kristallines Pulver | weißes, kristallines Pulver | weißes, kristallines Pulver mit Agglomeraten |
| **4 Wochen** | unverändert | weißes Pulver, Agglomerate | weißes, klebriges Pulver | weißes, klebriges Pulver | gelbes, klebriges Pulver | unverändert |
| **8 Wochen** | unverändert | weißes Pulver, Agglomerate | weißes, klebriges Pulver | weißes, klebriges Pulver | gelbes, klebriges Pulver | Unverändert |
| **12 Wochen** | unverändert | weißes Pulver, Agglomerate | weißes, klebriges Pulver | weißes, klebriges Pulver | gelbes, klebriges Pulver | Unverändert |

Chemische Reinheit und Wassergehalt blieben bei allen Proben mit Ausnahme des Phosphats unverändert. Beim Rasagilin-Phosphat stieg der Wassergehalt von 0,1% (t₀) über 0,2% (4 Wochen) auf 0,4% (8 Wochen) und der Gesamtgehalt an chemischen Verunreinigungen von 0,0% (t₀) über 0,05% (4 Wochen) auf 0,19% (8 Wochen). Die Werte nach 12 Wochen Lagerung wurden beim Rasagilin-Phosphat nicht mehr bestimmt.

### Beispiel 6

### Formulierungen

Gemäß folgender Tabelle wurde mit verschiedenen Rasagilinsalzen Tabletten durch zwei unterschiedliche Granulationsverfahren und durch ein Direktverpressungsverfahren hergestellt:

| **Formulierungen** | | | | | |
|---|---|---|---|---|---|
| **Zusammensetzung** | | *Granulation 1 mit Mannit* | | *Granulation 2 und Direktverpressen ohne Mannit* | |
| | | **[mg]** | **[%]** | **[mg]** | **[%]** |
| Rasagilin (als verschiedene Salze) | | 1 | *0,50* | 1 | *0,50* |
| | *API* | | | | |
| Mannit (Pearlitol 160 C) | | 109,00 | *54,23* | - | - |
| | *Füllstoff* | | | | |
| Avicel PH 101 | | 75,80 | *37,71* | 184,8 | *91,94* |
| | *Füllstoff* | | | | |
| Starch 1500 (vorverkleisterte Maisstarke) | | 10,00 | *4,98* | 10,00 | *4,98* |
| | *Sprengmittel* | | | | |
| Aerosil R972 (kolloidales, wasserfreies Siliciumdioxid) | | 1,20 | *0, 60* | 1,20 | *0, 60* |
| | *Gleitmittel* | | | | |
| Zitronensäure | | 2,00 | *1,00* | 2,00 | *1,00* |
| | *Stabilisator* | | | | |
| Magnesiumstearat | | 2,00 | *1,00* | 2,00 | *1,00* |
| | *Schmiermittel* | | | | |
| **Gesamt** | | **201,0** | ***100,0*** | **201,0** | ***100,0*** |

1. Naßgranulation (1000 Tabletten)

| | |
|---|---|
| 1. | Lösen von 2 g Zitronensäure und Rasagilinsalz in 40 g demineralisiertem Wasser in einem 100 ml Becherglas |
| 2. | Zugabe von 109 g Mannit (Pearlitol 160 C), 35 g Avicel PH 101 (Teil 1) und 10 g Starch 1500 in den Diosna P1 (Granulator-Hacker 1500 UpM, Rotor 500 UpM) und 3-minütiges Mischen |
| 3. | 2-Minütige Granulierung des Gemischs (Punkt 2) mit Granulationslösung (Punkt 1) in dem Diosna P1 (Hacker 1500 UpM, Rotor 500 UpM) |
| 4. | Granulierung des Gemischs (Punkt 3) in dem Diosna P1 für weitere 2 Minuten |
| 5. | Sieben des Granulats durch ein 2 mm Handsieb |
| 6. | Trocknen des Granulats über Nacht bei Raumtemperatur |
| 7. | Sieben des trockenen Granulats durch ein 0,3 mm Handsieb und Einfüllen in ein braunes 1-Liter-Glas |
| 8. | Zugabe von 40,8 g Avicel PH 101 (Teil 2) und 1,2 g Aerosil R972 in das braune Glas und 10-minütiges Mischen in einem Turbula-Mixer (30 UpM) |
| 9. | Zugabe von 2 g Magnesiumstearat zu dem Gemisch und 3-minütiges Mischen in dem Turbula (30 UpM) |
| 10. | Komprimieren der Tabletten mit 8 mm Stempel mit einer Excenter-Presse (Korsch EK0) |

2. Naßgranulation (1000 Tabletten)

| | |
|---|---|
| 1. | Lösen von 2 g Zitronensäure und Rasagilinsalz in 40 g demineralisiertem Wasser in einem 100 ml Becherglas |
| 2. | Zugabe von 92,4 g Avicel PH 101 (Teil 1) und 10 g Starch 1500 in den Diosna P1 (Granulator-Hacker 1500 UpM, Rotor 500 UpM) und 3-minütiges Mischen |
| 3. | 2-Minütige Granulierung des Gemischs (Punkt 2) mit Granulationslösung (Punkt 1) in dem Diosna P1 (Hacker 1500 UpM, Rotor 500 UpM) |
| 4. | Granulierung des Gemischs (Punkt 3) in dem Diosna P1 für weitere 2 Minuten |
| 5. | Sieben des Granulats durch ein 2 mm Handsieb |
| 6. | Trocknen des Granulats über Nacht bei Raumtemperatur |
| 7. | Sieben des trockenen Granulats durch ein 0,3 mm Handsieb und Einfüllen in ein braunes 1-Liter-Glas |
| 8. | Zugabe von 92,4 g Avicel PH 101 (Teil 2) und 1,2 g Aerosil R972 in das braune Glas und 10-minütiges Mischen in einem Turbula-Mixer (30 UpM) |
| 9. | Zugabe von 2 g Magnesiumstearat zu dem Gemisch und 3-minütiges Mischen in dem Turbula (30 UpM) |
| 10. | Komprimieren der Tabletten mit 8 mm Stempel mit einer Excenter-Presse (Korsch EK0) |

3. Direktverpressung (1000 Tabletten)

| | |
|---|---|
| 1. | Zugabe von Rasagilinsalz, 2 g Zitronensäure (mikronisiert) und 20 g Avicel PH 101 (Teil 1) in ein braunes 500 ml-Glas und 5-minütiges Mischen mit einem Turbula-Mixer (30 UpM) |
| 2. | Sieben des Gemischs (Punkt 1) durch ein 0,5 mm Handsieb |
| 3. | Zugabe von 10 g Starch 1500 und 70 g Avicel PH 101 (Teil 2) zu dem Gemisch (Punkt 2) und 5-minütiges Mischen in einem Turbula-Mixer (30 UpM) |
| 4. | Füllen des Gemischs aus Punkt 3 in ein braunes 1-Liter-Glas und Zugabe von 94,8 g Avicel PH 101 (Teil 3) in das braune Glas und 5-minütiges Mischen in einem Turbula-Mixer (30 UpM) |
| 5. | Zugabe von 1,2 g Aerosil R972 in das braune Glas und 5-minütiges Mischen in einem Turbula-Mixer (30 UpM) |
| 6. | Sieben des Pulvers durch ein 0,5 mm Handsieb und 5-minütiges Mischen in einem Turbula-Mixer (30 UpM) |
| 7. | Zugabe von 2 g Magnesiumstearat zu dem Gemisch und 5-minütiges Mischen in einem Turbula-Mixer (30 UpM) |
| 8. | Komprimieren der Tabletten mit 8 mm Stempel mit einer Excenter-Presse (Korsch EK0) |

Die Ergebnisse der verschiedenen Tablettierungen können der folgenden Tabelle entnommen werden:

| **Ergebnisse der Tablettierungen** | | | | | | |
|---|---|---|---|---|---|---|
| **Formulierung** | **Parameter** | **Salze** | | | | |
| | | Rasagilin-Mesylat | Rasagilin-Hydrochlorid | Rasagilin-Ethandisulfonat | Rasagilin-Phosphat | Rasagilin-Tartrat |
| Granulation 1 mit Mannit (55%) | Tablettengewicht | 201,1 mg | 199,7 mg | 201,0 mg | 205,3 mg | 203,7 mg |
| | Löslichkeit in Zitronensäurelösung | sehr hoch | sehr hoch | sehr hoch | hoch | hoch |
| | Härte bei 8 kN Presskraft | 54 N | 44 N | 57 N | 60 N | 61 N |
| Granulation 2 ohne Mannit | Tablettengewicht | 200,4 mg | 197,0 mg | 201,4 mg | 203,4 mg | 201,7 mg |
| | Löslichkeit in Zitronensäurelösung | sehr hoch | sehr hoch | sehr hoch | hoch | hoch |
| | Presskraft / Härte | 2,7 kN / 77,1 N | 2,7 kN / 61,8 N | 2,8 kN / 77,7 N | 2,8 kN / 75,2 N | 2,7 kN / 65,6 N |
| Direktverpressung ohne Mannit | Tablettengewicht | 201,3 mg | 199,8 mg | 201,0 mg | 201,0 mg | 201,0 mg |
| | Härte / RSD bei 2,7 kN Kompressionskraft | 77 N 14,10% | 74,4 N / 3,91% | 76,5 N / 2,90% | 72,6 N / 4,66% | 76,1 N / 4,73% |

### Beispiel 7

### Stabilitätstests

Die mit den verschiedenen Rasagilinsalzen hergestellten Tabletten des Beispiels 6 wurden Stabilitätstests bei 60°C in einer geschlossenen Glasflasche unterzogen. Die Ergebnisse können der folgenden Tabelle entnommen werden.

Lagerung bei 60°C in geschlossener Glasflasche, angegeben ist der Anteil an zersetztem Wirkstoff

| **Formulierung** | **Testpunkt** | **Mesylat** | **Hydrochlorid** | **Ethandisulfonat** | **Phosphat** | **Tartrat** |
|---|---|---|---|---|---|---|
| Granulation 1 mit Mannit (55%) | Ausgangswert | 0,00% | 0,00% | 0.00% | 0.00% | 0,00% |
| | 4 Wochen | 2,6% | 2,4% | 1,4% | 3,0% | 3,1% |
| Granulation 2 ohne Mannit | Ausgangswert | 0,00% | 0,00% | 0,00% | 0,00% | 0,00% |
| | 4 Wochen | 1,3% | 1,9% | 0,79% | 2,5% | 2,8% |
| Direktverpressung | Ausgangswert | 0,00% | 0,00% | 0,00% | 0,00% | 0,00% |
| ohne Mannit | 4 Wochen | 0,46% | 1,5% | 0,40% | 2,9% | 2,5% |

### Beispiel 8

### Teilchengrößenverteilung

Rasagilin-Edisilat (Rasagilin-EDS) wurde wie in Beispiel 1 beschrieben hergestellt, Rasagilin-Oxalat wie in Beispiel 2. Analog zu Beispiel 1 wurde ebenfalls das Rasagilin-Tartrat und das Rasagilin-Mesylat hergestellt.

Die Partikelgrößenanalysen wurde mittels Laserbeugung in einem Malvern Mastersizer 2000 mit Hydro 2000S Nassdispergiereinheit durchgeführt. Die Plausibilität der Ergebnisse wurde lichtmikroskopisch mit einem Leica Z 16 APO überprüft.

Das Prinzip der Partikelgrößenmessung durch Laserbeugung beruht auf der Tatsache, dass der Beugungswinkel von Licht an einem Partikel umgekehrt proportional zur Größe des Partikels ist. Das Idealisierte Modell eines Partikels ist hierbei kugelförmig, da die Kugel das einzige dreidimensionale Gebilde ist, dessen Größe in zweidimensionaler Projektion durch einen einzigen Parameter eindeutig bestimmt ist.

Anerkannte Kenngrößen der Partikelgrößenverteilung sind die Werte d(0.1), d(0.5) und d(0.9). d(0.1) ist das 0,1-Quantil der Verteilung. Demzufolge sind 10% (volumengewichtet) der Partikel eines Gemisches kleiner als der für d(0.1) angegebene Wert. Entsprechend sind d(0.5) und d(0.9) das 0,5- bzw. 0,9-Quantil und somit 50 % bzw. 90 % der Partikel (volumengewichtet) kleiner als der jeweils angegebene Wert.

Alle Messungen wurden bei einer Rührgeschwindigkeit von 2500 U/min durchgeführt. Die Hintergrund- und die Messzeit betrugen jeweils 10 s. Die Probenkonzentration wurde so gewählt, dass die Abschwächung des Laserlichts (Obscuration) bei 10 bis 20% lag. Als Dispergiermittel diente dünnflüssiges Paraffin.

Die folgenden Parameter wurden für die verschiedenen Salze individuell gewählt:

| **Probe** | **Beschallungs-zeit [min]** | **Beschallungs-intensität [%]** | **Beschallung während der Messung** | **nur Rotlicht** |
|---|---|---|---|---|
| Rasagilin-Oxalat | 10 | 30 | nein | nein |
| Rasagilin-Tartrat | 2 | 50 | ja | ja |
| Rasagilin-EDS | 2 | 30 | nein | nein |
| Rasagilin-Mesylat | 5 | 30 | nein | nein |

### Ergebnisse:

Dargestellt sind die Mittelwerte aus drei Messzyklen ± Standardabweichung (SD)

| **Salz** | **d(0.1) ± SD** **[µm]** | **d(0.5) ± SD** **[µm]** | **d(0.9) ± SD** **[µm]** | **Anteil ≤ 250 µm** |
|---|---|---|---|---|
| Rasagilin-Oxalat | 19,9 ± 0,02 | 53,0 ± 0,09 | 120,5 ± 0,66 | 98,1 % |
| Rasagilin-Tartrat | 5,0 ± 0,04 | 40,9 ± 0,57 | 161,2 ± 1,34 | 97,2 % |
| Rasagilin-EDS | 2,7 ± 0,01 | 12,8 ± 0,08 | 35,1 ± 0,36 | 100,0 % |
| Rasagilin-Mesylat | 13,6 ± 0,05 | 40,5 ± 0,25 | 85,7 ± 0,89 | 100,0 % |

## Patentansprüche

1. Salz des Rasagilins mit einer Säure der allgemeinen Formel I 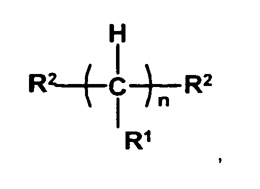 in der die Reste R¹ unabhängig voneinander ein Wasserstoffatom oder einen C₁-C₄-Alkylrest darstellen,
R² eine COOH-Gruppe oder eine S(O)ₘH-Gruppe darstellt,
m die Zahl 2, 3 oder 4 ist und
n eine ganze Zahl im Bereich von 0 bis 4 darstellt.

2. Salz des Rasagilins nach Anspruch 1, wobei alle Reste R¹ ein Wasserstoffatom darstellen.

3. Salz des Rasagilins nach Anspruch 1 oder 2, wobei n 0 oder 2 ist.

4. Salz des Rasagilins nach Anspruch 3, ausgewählt aus dem Rasagilin-Edisilat und dem Rasagilin-Oxalat.

5. Arzneimittel enthaltend ein Salz des Rasagilins nach einem der Ansprüche 1 bis 4.

6. Arzneimittel nach Anspruch 5, **dadurch gekennzeichnet, dass** es eine Tablette darstellt.

7. Verfahren zur Stabilisierung von Rasagilin in einem ein Rasagilinsalz enthaltenden Arzneimittel, **dadurch gekennzeichnet, dass** als Rasagilinsalz ein Salz des Rasagilins nach einem der Ansprüche 1 bis 4 eingesetzt wird.

## Claims

1. Salt of rasagiline having an acid of general formula I 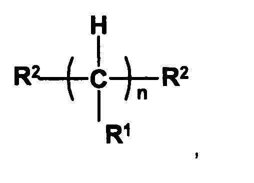 wherein the residues R¹ are independently a hydrogen atom or a C₁-C₄ alkyl residue,
R² is a COOH group or an S(O)ₘH group,
m is the value 2, 3 or 4 and
n is an integer ranging from 0 to 4.

2. Salt of rasagiline according to claim 1, wherein all residues R¹ represent a hydrogen atom.

3. Salt of rasagiline according to claim 1 or 2, wherein n is 0 or 2.

4. Salt of rasagiline according to claim 3, selected from the rasagiline edisilate and the rasagiline oxalate.

5. Pharmaceutical preparation containing a salt of rasagiline according to any of claims 1 to 4.

6. Pharmaceutical preparation according to claim 5, **characterized in that** it represents a tablet.

7. Process for stabilizing rasagiline in a pharmaceutical preparation containing a rasagiline salt, **characterized in that** a salt of rasagiline according to any of claims 1 to 4 is used as the rasagiline salt.

## Revendications

1. Sel de rasagiline, obtenu avec un acide de formule générale I : 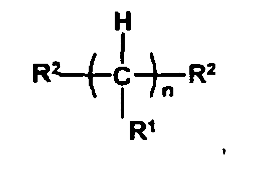 dans laquelle les radicaux R¹ représentent, indépendamment l'un de l'autre, un atome d'hydrogène ou un radical alkyle en C₁-C₄
R² représente un groupement COOH ou un groupement S(O)ₘH,
m est le nombre 2, 3 ou 4, et
n est un nombre entier situé dans la plage de 0 à 4.

2. Sel de rasagiline selon la revendication 1, dans lequel tous les radicaux R¹ représentent un atome d'hydrogène.

3. Sel de rasagiline selon la revendication 1 ou 2, dans lequel n est égal à 0 ou à 2.

4. Sel de rasagiline selon la revendication 3, choisi parmi l'édisylate de rasagiline et l'oxalate de rasagiline.

5. Médicament contenant un sel de rasagiline selon l'une quelconque des revendications 1 à 4.

6. Médicament selon la revendication 5, **caractérisé en ce qu'**il représente un comprimé.

7. Procédé de stabilisation de la rasagiline dans un médicament contenant un sel de rasagiline, **caractérisé en ce que** l'on utilise, comme sel de rasagiline, un sel de rasagiline selon l'une quelconque des revendications 1 à 4.
